# EUROPEAN PATENT APPLICATION

(11) **EP 4 835 065 A1**
(43) Date of publication of application: **04.11.2026**
(21) Application number: 24912736.6
(22) Date of filing: 23.12.2024
(51) Int. Cl.: C12N 9/12, A61K 31/7105, A61K 48/00, A61P 43/00, C12N 1/15, C12N 1/19, C12N 1/21, C12N 5/10, C12N 15/10, C12N 15/54, C12N 15/63, C12P 19/34, C12P 21/02

(54) **MODIFIED RNA POLYMERASE**

(30) Priority: 26.12.2023 JP 2023220132; 24.10.2024 JP 2024187422
(71) Applicant: Toyobo Co., Ltd., Kita-ku Osaka-shi, Osaka 530-0001 (JP)
(72) Inventor: OCHI, Kensuke, Tsuruga-shi, Fukui 914-8550 (JP)
(74) Representative: Vossius & Partner Patentanwälte Rechtsanwälte mbB
(86) International application number: PCT/JP2024/045489
(87) International publication number: WO 2025/142849

(57) **Abstract**

An object is to provide a novel modified RNA polymerase. The object is achieved by a modified RNA polymerase in which at least one amino acid at a predetermined position is modified.

## Description

### Technical Field

The present invention relates to a modified RNA polymerase.

### Background Art

DNA-dependent RNA polymerase is an enzyme that recognizes promoter sequences in DNA and synthesizes RNA using DNA as a template, and has been the subject of extensive research because it is responsible for the transcription reaction that synthesizes mRNA from genomic DNA in living organisms. In particular, phage-derived RNA polymerases have a relatively simple structure and function as a single subunit, and have been the subject of biochemical and structural biological research since long ago. These RNA polymerases are also widely used for the purpose of synthesizing RNA in vitro, and the RNA thus synthesized is utilized as RNA probes for use in molecular biological methods, as various functional RNAs, or as template RNAs for protein expression in cells or for use in cell-free protein synthesis systems.

Furthermore, isothermal amplification reaction techniques such as NASBA and TMA have been developed by using the ability of RNA polymerase to synthesize numerous RNA molecules from a template DNA, and have been applied to clinical diagnostics.

In recent years, RNA synthesized by an RNA polymerase has been applied to mRNA therapeutics, including mRNA vaccines. RNA polymerases have also been widely used in the field of pharmaceutical production. One impurity that is problematic in the production process of mRNA therapeutics is dsRNA. Since dsRNA induces inflammatory responses in living organisms, it is desirable to remove it to the greatest extent possible from mRNA therapeutics. NPL 1 discloses a modified T7 RNA polymerase capable of reducing the generation of dsRNA in a transcription reaction.

### Citation List

### Non-patent Literature

NPL 1: Dousis, A., Ravichandran, K., Hobert, E.M. et al., Nat. Biotechnol., 41:560-568, 2023

### Summary of Invention

### Technical Problem

As described above, as the applications of RNA polymerase expand, further improvements in the performance of RNA polymerase are desired. Thus, the present invention aims to provide a novel modified RNA polymerase.

### Solution to Problem

As a result of extensive study to achieve the aim described above, the present inventors found that when at least one amino acid at a predetermined position in the amino acid sequence of a wild-type RNA polymerase is modified, it is possible to reduce the generation of dsRNA in a transcription reaction. The present inventors further conducted extensive study based on the above finding, and as a result, completed the present invention.

Specifically, the present invention encompasses the following aspects.
Item 1
   An RNA polymerase comprising an amino acid sequence having 90% or more sequence identity to the amino acid sequence of SEQ ID NO: 1, wherein at least one selected from the group consisting of amino acids at positions corresponding to positions 61, 156, 189, 165, and 661 of the amino acid sequence of SEQ ID NO: 1 is modified.
Item 2
   An RNA polymerase comprising an amino acid sequence having 90% or more sequence identity to the amino acid sequence of SEQ ID NO: 1, wherein at least one selected from the group consisting of the following (i) to (v) is satisfied:
   (i) an amino acid at a position corresponding to position 61 of the amino acid sequence of SEQ ID NO: 1 is glutamic acid, aspartic acid, serine, glycine, or proline;
   (ii) an amino acid at a position corresponding to position 156 of the amino acid sequence of SEQ ID NO: 1 is asparagine;
   (iii) an amino acid at a position corresponding to position 189 of the amino acid sequence of SEQ ID NO: 1 is asparagine;
   (iv) an amino acid at a position corresponding to position 165 of the amino acid sequence of SEQ ID NO: 1 is serine, glutamic acid, arginine, or proline; and
   (v) an amino acid at a position corresponding to position 661 of the amino acid sequence of SEQ ID NO: 1 is asparagine.
Item 3 The RNA polymerase according to Item 1 or 2, comprising an amino acid sequence having 90% or more sequence identity to the amino acid sequence of SEQ ID NO: 1, wherein
   (1) an amino acid at a position corresponding to position 61 of the amino acid sequence of SEQ ID NO: 1 is glutamic acid, aspartic acid, serine, glycine, or proline;
   (2) an amino acid at a position corresponding to position 156 of the amino acid sequence of SEQ ID NO: 1 is asparagine;
   (3) an amino acid at a position corresponding to position 189 of the amino acid sequence of SEQ ID NO: 1 is asparagine;
   (4) an amino acid at a position corresponding to position 165 of the amino acid sequence of SEQ ID NO: 1 is serine, glutamic acid, arginine, or proline;
   (5) an amino acid at a position corresponding to position 661 of the amino acid sequence of SEQ ID NO: 1 is asparagine;
   (6) an amino acid at a position corresponding to position 61 of the amino acid sequence of SEQ ID NO: 1 is glutamic acid, and an amino acid at a position corresponding to position 156 of the amino acid sequence of SEQ ID NO: 1 is asparagine;
   (7) an amino acid at a position corresponding to position 61 of the amino acid sequence of SEQ ID NO: 1 is glutamic acid, and an amino acid at a position corresponding to position 165 of the amino acid sequence of SEQ ID NO: 1 is serine;
   (8) an amino acid at a position corresponding to position 61 of the amino acid sequence of SEQ ID NO: 1 is glutamic acid, and an amino acid at a position corresponding to position 189 of the amino acid sequence of SEQ ID NO: 1 is asparagine;
   (9) an amino acid at a position corresponding to position 61 of the amino acid sequence of SEQ ID NO: 1 is glutamic acid, and an amino acid at a position corresponding to position 661 of the amino acid sequence of SEQ ID NO: 1 is asparagine;
   (10) an amino acid at a position corresponding to position 61 of the amino acid sequence of SEQ ID NO: 1 is proline, and an amino acid at a position corresponding to position 165 of the amino acid sequence of SEQ ID NO: 1 is serine;
   (11) an amino acid at a position corresponding to position 61 of the amino acid sequence of SEQ ID NO: 1 is proline, and an amino acid at a position corresponding to position 189 of the amino acid sequence of SEQ ID NO: 1 is asparagine;
   (12) an amino acid at a position corresponding to position 61 of the amino acid sequence of SEQ ID NO: 1 is proline, and an amino acid at a position corresponding to position 661 of the amino acid sequence of SEQ ID NO: 1 is asparagine;
   (13) an amino acid at a position corresponding to position 61 of the amino acid sequence of SEQ ID NO: 1 is glutamic acid, an amino acid at a position corresponding to position 156 of the amino acid sequence of SEQ ID NO: 1 is asparagine, and an amino acid at a position corresponding to position 165 of the amino acid sequence of SEQ ID NO: 1 is serine;
   (14) an amino acid at a position corresponding to position 61 of the amino acid sequence of SEQ ID NO: 1 is glutamic acid, an amino acid at a position corresponding to position 165 of the amino acid sequence of SEQ ID NO: 1 is serine, and an amino acid at a position corresponding to position 189 of the amino acid sequence of SEQ ID NO: 1 is asparagine;
   (15) an amino acid at a position corresponding to position 61 of the amino acid sequence of SEQ ID NO: 1 is glutamic acid, an amino acid at a position corresponding to position 165 of the amino acid sequence of SEQ ID NO: 1 is serine, and an amino acid at a position corresponding to position 661 of the amino acid sequence of SEQ ID NO: 1 is asparagine;
   (16) an amino acid at a position corresponding to position 61 of the amino acid sequence of SEQ ID NO: 1 is proline, an amino acid at a position corresponding to position 156 of the amino acid sequence of SEQ ID NO: 1 is asparagine, and an amino acid at a position corresponding to position 165 of the amino acid sequence of SEQ ID NO: 1 is serine;
   (17) an amino acid at a position corresponding to position 61 of the amino acid sequence of SEQ ID NO: 1 is proline, an amino acid at a position corresponding to position 165 of the amino acid sequence of SEQ ID NO: 1 is serine, and an amino acid at a position corresponding to position 189 of the amino acid sequence of SEQ ID NO: 1 is asparagine;
   (18) an amino acid at a position corresponding to position 61 of the amino acid sequence of SEQ ID NO: 1 is proline, an amino acid at a position corresponding to position 165 of the amino acid sequence of SEQ ID NO: 1 is serine, and an amino acid at a position corresponding to position 661 of the amino acid sequence of SEQ ID NO: 1 is asparagine;
   (19) an amino acid at a position corresponding to position 61 of the amino acid sequence of SEQ ID NO: 1 is glutamic acid, an amino acid at a position corresponding to position 156 of the amino acid sequence of SEQ ID NO: 1 is asparagine, an amino acid at a position corresponding to position 165 of the amino acid sequence of SEQ ID NO: 1 is serine, and an amino acid at a position corresponding to position 189 of the amino acid sequence of SEQ ID NO: 1 is asparagine;
   (20) an amino acid at a position corresponding to position 61 of the amino acid sequence of SEQ ID NO: 1 is glutamic acid, an amino acid at a position corresponding to position 165 of the amino acid sequence of SEQ ID NO: 1 is serine, an amino acid at a position corresponding to position 189 of the amino acid sequence of SEQ ID NO: 1 is asparagine, and an amino acid at a position corresponding to position 661 of the amino acid sequence of SEQ ID NO: 1 is asparagine;
   (21) an amino acid at a position corresponding to position 61 of the amino acid sequence of SEQ ID NO: 1 is proline, an amino acid at a position corresponding to position 156 of the amino acid sequence of SEQ ID NO: 1 is asparagine, an amino acid at a position corresponding to position 165 of the amino acid sequence of SEQ ID NO: 1 is serine, and an amino acid at a position corresponding to position 189 of the amino acid sequence of SEQ ID NO: 1 is asparagine;
   (22) an amino acid at a position corresponding to position 61 of the amino acid sequence of SEQ ID NO: 1 is proline, an amino acid at a position corresponding to position 165 of the amino acid sequence of SEQ ID NO: 1 is serine, an amino acid at a position corresponding to position 189 of the amino acid sequence of SEQ ID NO: 1 is asparagine, and an amino acid at a position corresponding to position 661 of the amino acid sequence of SEQ ID NO: 1 is asparagine;
   (23) an amino acid at a position corresponding to position 61 of the amino acid sequence of SEQ ID NO: 1 is glutamic acid, an amino acid at a position corresponding to position 156 of the amino acid sequence of SEQ ID NO: 1 is asparagine, an amino acid at a position corresponding to position 165 of the amino acid sequence of SEQ ID NO: 1 is serine, an amino acid at a position corresponding to position 189 of the amino acid sequence of SEQ ID NO: 1 is asparagine, and an amino acid at a position corresponding to position 661 of the amino acid sequence of SEQ ID NO: 1 is asparagine; or
   (24) an amino acid at a position corresponding to position 61 of the amino acid sequence of SEQ ID NO: 1 is proline, an amino acid at a position corresponding to position 156 of the amino acid sequence of SEQ ID NO: 1 is asparagine, an amino acid at a position corresponding to position 165 of the amino acid sequence of SEQ ID NO: 1 is serine, an amino acid at a position corresponding to position 189 of the amino acid sequence of SEQ ID NO: 1 is asparagine, and an amino acid at a position corresponding to position 661 of the amino acid sequence of SEQ ID NO: 1 is asparagine.
Item 4
   A polynucleotide encoding the RNA polymerase according to any one of Items 1 to 3.
Item 5
   A vector comprising the polynucleotide according to Item 4.
Item 6
   A recombinant host cell transformed with the vector according to Item 5.
Item 7
   A method for producing an RNA polymerase using the polynucleotide according to Item 4, the vector according to Item 5, and/or the recombinant host cell according to Item 6.
Item 8
   A reagent comprising the RNA polymerase according to any one of Items 1 to 3, the polynucleotide according to Item 4, the vector according to Item 5, and/or the recombinant host cell according to Item 6.
Item 9
   An RNA synthesis method comprising allowing the RNA polymerase according to any one of Items 1 to 3 to act on a template DNA.
Item 10
   The method according to Item 9, wherein the proportion of dsRNA in the synthesized RNA is less than 90% relative to the proportion of dsRNA in RNA synthesized using a corresponding wild-type RNA polymerase under the same conditions.
Item 11
   The method according to Item 9 or 10, wherein the amount of the synthesized RNA is 110% or more relative to the amount of RNA synthesized using a corresponding wild-type RNA polymerase under the same conditions.
Item 12
   The method according to any one of Items 9 to 11, wherein the RNA synthesis is mRNA synthesis.
Item 13
   The method according to Item 12, wherein the proportion of dsRNA in the synthesized mRNA is less than 90% relative to the proportion of dsRNA in mRNA synthesized using a corresponding wild-type RNA polymerase under the same conditions.
Item 14
   The method according to Item 12 or 13, wherein the amount of the synthesized mRNA is 110% or more relative to the amount of mRNA synthesized using a corresponding wild-type RNA polymerase under the same conditions.
Item 15
   The method according to any one of Items 9 to 11, wherein the RNA synthesis is non-coding RNA synthesis.
Item 16
   The method according to Item 15, wherein the non-coding RNA is at least one selected from the group consisting of microRNA, siRNA, piRNA, rRNA, tRNA, snRNA, snoRNA, SLRNA, SRPRNA, mRNA-like non-coding RNA, and oligonucleotide.
Item 17
   A method for synthesizing guide RNA for gene editing, using the RNA polymerase according to any one of Items 1 to 3.
Item 18
   A method for synthesizing an RNA therapeutic using the RNA polymerase according to any one of Items 1 to 3.
Item 19
   The method according to Item 9, wherein the RNA synthesis is performed by an isothermal amplification reaction.
Item 20
   A method for performing genetic testing using the RNA polymerase according to any one of Items 1 to 3.
Item 21
   A method for performing in vivo protein expression or in vitro cell-free protein synthesis using the RNA polymerase according to any one of Items 1 to 3.

### Advantageous Effects of Invention

The present invention can provide a novel modified RNA polymerase.

### Description of Embodiments

The present invention will be described below in further detail with reference to the embodiments of the present invention; however, the present invention is not limited thereto.

All NPLs and PTLs described herein are incorporated herein by reference in their entirety. As used herein, "to" means "from X to Y, inclusive," and, for example, when "X to Y" is described herein, it means "X or more and Y or less." As used herein, "and/or" means any one of the listed elements or any possible combination of two or more thereof. As used herein, the terms "include," "contain," and variations thereof express concepts that encompass all of "comprise," "consist essentially of," and "consist of."

In one aspect, the present invention provides a modified RNA polymerase. In the present specification, the term "modification" refers to mutation of an amino acid residue, i.e., substitution, deletion, insertion, or addition. The modified RNA polymerase (also referred to below as a "modified RNA polymerase") refers to one in which at least one amino acid residue in the amino acid sequence of a wild-type RNA polymerase is mutated, i.e., substituted, deleted, inserted, or added.

In the present specification, the term "wild-type RNA polymerase" (also referred to below as "wild-type" or "WT") refers to an RNA polymerase having no artificially introduced mutations. Examples of wild-type RNA polymerases include T7 RNA polymerase, T3 RNA polymerase, K11 RNA polymerase, SP6 RNA polymerase, Syn5 RNA polymerase, KP34 RNA polymerase, and VSW-3 RNA polymerase. A preferred wild-type RNA polymerase may be, for example, wild-type T7 RNA polymerase consisting of the amino acid sequence of SEQ ID NO: 1. As is well known to those skilled in the art, T7 RNA polymerase means an RNA polymerase derived from T7 phage.

The RNA polymerase of the present invention can exhibit improved specific activity, as compared to a corresponding wild-type RNA polymerase. Here, the term "specific activity" refers to the enzymatic activity per unit protein mass, as measured at a temperature of 20°C to 100°C, for example. The term "corresponding wild-type RNA polymerase" refers to the wild-type RNA polymerase of the same type as a given modified RNA polymerase. For example, the "corresponding wild-type RNA polymerase" for a modified T7 RNA polymerase is wild-type T7 RNA polymerase. In addition, for example, the "corresponding wild-type RNA polymerase" for a modified T3 RNA polymerase is wild-type T3 RNA polymerase.

The RNA polymerase of the present invention comprises an amino acid sequence different from that of a conventionally known wild-type RNA polymerase. Thus, in the present specification, the RNA polymerase of the present invention is sometimes referred to as "the mutant RNA polymerase" or "the modified RNA polymerase." In the present specification, the terms "mutant" and the "modified" respectively in the "mutant RNA polymerase" and the "modified RNA polymerase" are used interchangeably, and these terms refer to an RNA polymerases having an amino acid sequence that differs from that of a conventionally known wild-type RNA polymerase, without distinguishing whether the difference arises from an artificial mutation or a naturally occurring mutation. Thus, the mutant RNA polymerase of the present invention is an RNA polymerase having an amino acid sequence different from the amino acid sequence of SEQ ID NO: 1, wherein at least one amino acid in the amino acid sequence of SEQ ID NO: 1 representing a wild-type T7 RNA polymerase sequence is modified. The mutant RNA polymerase is not limited as to whether it is an RNA polymerase obtained by an artificial mutation or an RNA polymerase obtained by naturally occurring mutation.

In the present specification, simplified symbols using alphabetical notation may be used for nucleotide sequences, amino acid sequences, and their individual components, all of which follow conventions in the fields of molecular biology and genetic engineering. In addition, in the present specification, notations such as "A61E" are used to concisely indicate mutations in amino acid sequences. "A61E" indicates substitution of alanine (A) at position 61 of the amino acid sequence with glutamic acid (E). That is, it indicates the type of amino acid residue before substitution, the position of the substituted amino acid in the amino acid sequence, and the type of amino acid residue after substitution. In addition, the sequence numbers correspond to the sequence numbers listed in the sequence listing, unless otherwise specified. In the case of multiple mutants, the above notations can be connected with "/" (for example, it can be described as A61E/D156N/N165S).

In one embodiment, the amino acid sequence of the mutant RNA polymerase may have modifications at a certain proportion of the amino acid sequence of SEQ ID NO: 1. In one embodiment, preferably, the amino acid sequence of the mutant RNA polymerase has 90% or more sequence identity to the amino acid sequence of SEQ ID NO: 1. Although it is not particularly limited as long as the RNA polymerase activity is not lost, the mutant RNA polymerase comprises an amino acid sequence having, for example, 90% or more, preferably 95% or more, 96% or more, 97% or more, 98% or more, or 99% or more sequence identity to the amino acid sequence of SEQ ID NO: 1. Here, the amino acid sequence identity can be evaluated by any means known in the art. For example, it can be calculated using commercially available analysis tools or analysis tools available via a telecommunication line (internet). For example, amino acid sequence identity can be calculated using the homology algorithm BLAST (Basic Local Alignment Search Tool) of the National Center for Biotechnology Information (NCBI) at http://www.ncbi.nlm.nih.gov/BLAST/ with default (initial) parameters at BLAST. Further, the amino acid sequence of the mutant RNA polymerase may be an amino acid sequence in which one or more amino acids are deleted, substituted, inserted, and/or added in the amino acid sequence of SEQ ID NO: 1. Here, "one or more" may mean, for example, 1 to 30, preferably 1 to 20, 1 to 10, 1 to 5, or 1 to 3, although it is not particularly limited as long as the RNA polymerase activity is not lost. Such an amino acid sequence may be one artificially produced by a genetic engineering technique, or may be an amino acid sequence of a naturally derived protein.

In one embodiment, when a mutant RNA polymerase has a substitution in the amino acid sequence of SEQ ID NO: 1, the substitution is preferably a substitution between structurally and/or chemically similar amino acids (so-called conservative substitution). Non-limiting examples of conservative substitutions include substitutions between basic amino acids (H, K, R), substitutions between acidic amino acids (D, E), substitutions between neutral nonpolar amino acids (A, G, V, L, I, P, F, M, W), substitutions between neutral polar amino acids (N, Q, S, T, Y, C), substitutions between aromatic amino acids (W, F, H, Y), substitutions between nitrogen-containing amino acids (K, R, N, Q, P), substitutions between sulfur-containing amino acids (C, M), substitutions between oxygen-containing amino acids (S, T), substitutions between β-branched chain amino acids (V, L, I), and substitutions between amino acids having a linear alkyl or hydrogen side chain (A, G).

In a specific embodiment, the RNA polymerase of the present invention may comprise a peptide other than the RNA polymerase on either the N-terminal side or the C-terminal side, or both. Non-limiting examples of such peptides include extracellular secretion signals, protein purification tags (His tag, FLAG tag, Strep tag, GST tag, MBP tag, etc.), proteins having other functions, and linkers.

The RNA polymerase of the present invention may comprise an amino acid sequence including the amino acid residues described above. A person skilled in the art can produce an RNA polymerase protein having a target amino acid sequence by any genetic engineering technique known in the art, for example, by procedures including appropriately designing a base sequence encoding the target amino acid sequence, incorporating the base sequence into an appropriate expression vector or the like, and transforming a host cell with the resulting vector to allow expression.

In a preferred embodiment, the RNA polymerase of the present invention is an RNA polymerase comprising an amino acid sequence having, for example, 90% or more, preferably 95% or more, 96% or more, 97% or more, 98% or more, or 99% or more sequence identity to the amino acid sequence of SEQ ID NO: 1, wherein, for example, an amino acid at a position corresponding to position 61 of the amino acid sequence of SEQ ID NO: 1 is modified, and preferably, the amino acid at a position corresponding to position 61 is substituted with a different amino acid. The amino acid at a position corresponding to position 61 of the amino acid sequence of SEQ ID NO: 1 is alanine. Examples of amino acids that may be substituted for an alanine residue at a position corresponding to position 61 include glycine, proline, isoleucine, leucine, methionine, valine, phenylalanine, tryptophan, tyrosine, cysteine, asparagine, glutamine, serine, threonine, arginine, histidine, lysine, aspartic acid, and glutamic acid. Glutamic acid, aspartic acid, serine, threonine, glycine, and proline are preferred, and glutamic acid, aspartic acid, serine, glycine, and proline are more preferred. Without intending to be bound by any particular theory, an amino acid mutation at a position corresponding to position 61 of the amino acid sequence of SEQ ID NO: 1 is considered to contribute to reducing the proportion of dsRNA in RNA produced by a transcription reaction using the modified RNA polymerase.

In the present specification, the expression "a position corresponding to position 61 of the amino acid sequence of SEQ ID NO: 1" refers to the position corresponding to position 61 of the amino acid sequence of wild-type T7 RNA polymerase (SEQ ID NO: 1) or the position corresponding to position 61 of the amino acid sequence of wild-type T7 RNA polymerase (SEQ ID NO: 1) in the amino acid sequence of the mutant RNA polymerase. The "position corresponding to position 61 of the amino acid sequence of SEQ ID NO: 1" in the amino acid sequence of a mutant RNA polymerase can be readily identified by comparing and aligning the amino acid sequence of wild-type T7 RNA polymerase (SEQ ID NO: 1) with the amino acid sequence of the mutant RNA polymerase using any known method. Such comparison and alignment of amino acid sequences can be performed using commercially available analysis tools or tools available via telecommunication lines (internet). For example, amino acid sequence alignment can be performed using the multiple sequence alignment program Clustal Omega provided by the European Bioinformatics Institute (EMBL) (https://www.ebi.ac.uk/jdispatcher/msa/clustalo) with default (initial) parameters. In the present specification, the same applies to cases when a position corresponding to an amino acid at any position other than the position 61 of the amino acid sequence of SEQ ID NO: 1 is described.

In a preferred embodiment, the RNA polymerase of the present invention is an RNA polymerase comprising an amino acid sequence having, for example, 90% or more, preferably 95% or more, 96% or more, 97% or more, 98% or more, or 99% or more sequence identity to the amino acid sequence of SEQ ID NO: 1, wherein, for example, an amino acid at a position corresponding to position 156 of the amino acid sequence of SEQ ID NO: 1 is modified, preferably, by substitution with a different amino acid. The amino acid at a position corresponding to position 156 of the amino acid sequence of SEQ ID NO: 1 is aspartic acid. Non-limiting examples of amino acids that may be substituted for an aspartic acid residue at a position corresponding to position 156 include glycine, alanine, proline, isoleucine, leucine, methionine, valine, phenylalanine, tryptophan, tyrosine, cysteine, asparagine, glutamine, serine, threonine, arginine, histidine, lysine, and glutamic acid. In a more preferred embodiment, the RNA polymerase of the present invention is an RNA polymerase, wherein an amino acid at a position corresponding to position 156 of the amino acid sequence of SEQ ID NO: 1 is modified to a basic amino acid or a neutral amino acid. Examples of basic amino acids or neutral amino acids include asparagine, glycine, alanine, proline, isoleucine, leucine, valine, phenylalanine, tryptophan, tyrosine, glutamine, cysteine, methionine, serine, threonine, arginine, histidine, and lysine. Asparagine and glutamine are preferred, and asparagine is more preferred. Without intending to be bound by any particular theory, an amino acid mutation at a position corresponding to position 156 of the amino acid sequence of SEQ ID NO: 1 is considered to contribute to reducing the proportion of dsRNA in RNA produced by a transcription reaction using the modified RNA polymerase.

In a preferred embodiment, the RNA polymerase of the present invention is an RNA polymerase comprising an amino acid sequence having, for example, 90% or more, preferably 95% or more, 96% or more, 97% or more, 98% or more, or 99% or more sequence identity to the amino acid sequence of SEQ ID NO: 1, wherein, for example, an amino acid at a position corresponding to position 165 of the amino acid sequence of SEQ ID NO: 1 is modified, preferably by substitution with a different amino acid. The amino acid at a position corresponding to position 165 of the amino acid sequence of SEQ ID NO: 1 is asparagine. Examples of amino acids that may be substituted for an asparagine residue at a position corresponding to position 165 include glycine, alanine, proline, isoleucine, leucine, methionine, valine, phenylalanine, tryptophan, tyrosine, cysteine, glutamine, serine, threonine, arginine, histidine, lysine, aspartic acid, and glutamic acid. Serine, glutamic acid, arginine, and proline are preferred, and serine is more preferred. Without intending to be bound by any particular theory, an amino acid mutation at a position corresponding to position 165 of the amino acid sequence of SEQ ID NO: 1 is considered to contribute to reducing the proportion of dsRNA in RNA produced by a transcription reaction using the modified RNA polymerase.

In a preferred embodiment, the RNA polymerase of the present invention is an RNA polymerase comprising an amino acid sequence having, for example, 90% or more, preferably 95% or more, 96% or more, 97% or more, 98% or more, or 99% or more sequence identity to the amino acid sequence of SEQ ID NO: 1, wherein, for example, an amino acid at a position corresponding to position 189 of the amino acid sequence of SEQ ID NO: 1 is modified, preferably by substitution with a different amino acid. The amino acid at a position corresponding to position 189 of the amino acid sequence of SEQ ID NO: 1 is aspartic acid. Non-limiting examples of amino acids that may be substituted for aspartic acid residue at a position corresponding to position 189 include glycine, alanine, proline, isoleucine, leucine, methionine, valine, phenylalanine, tryptophan, tyrosine, cysteine, asparagine, glutamine, serine, threonine, arginine, histidine, lysine, and glutamic acid. In a more preferred embodiment, in the RNA polymerase of the present invention, the amino acid at a position corresponding to position 189 of the amino acid sequence of SEQ ID NO: 1 is preferably substituted with a basic amino acid or a neutral amino acid. Examples of basic amino acids or neutral amino acids include glycine, alanine, proline, isoleucine, leucine, valine, phenylalanine, tryptophan, tyrosine, asparagine, glutamine, cysteine, methionine, serine, threonine, arginine, histidine, and lysine. Substitution with asparagine or glutamine is preferred, and substitution with asparagine is more preferred. Without intending to be bound by any particular theory, an amino acid mutation at a position corresponding to position 189 of the amino acid sequence of SEQ ID NO: 1 is considered to contribute to reducing the proportion of dsRNA in RNA produced by a transcription reaction using the modified RNA polymerase.

In a preferred embodiment, the RNA polymerase of the present invention comprises an amino acid sequence having, for example, 90% or more, preferably 95% or more, 96% or more, 97% or more, 98% or more, or 99% or more sequence identity to the amino acid sequence of SEQ ID NO: 1, wherein, for example, an amino acid at a position corresponding to position 661 of the amino acid sequence of SEQ ID NO: 1 is modified, preferably by substitution with a different amino acid. The amino acid at a position corresponding to position 661 of the amino acid sequence of SEQ ID NO: 1 is serine. Examples of amino acids that may be substituted for serine at a position corresponding to position 661 of the amino acid sequence of SEQ ID NO: 1 include glycine, alanine, proline, isoleucine, leucine, methionine, valine, phenylalanine, tryptophan, tyrosine, cysteine, asparagine, glutamine, threonine, arginine, histidine, lysine, aspartic acid, and glutamic acid. Substitution with asparagine or glutamine is preferred, and substitution with asparagine is more preferred. Without intending to be bound by any particular theory, an amino acid mutation at a position corresponding to 661 of the amino acid sequence of SEQ ID NO: 1 is considered to contribute to reducing the proportion of dsRNA in RNA produced by a transcription reaction using the modified RNA polymerase.

In a preferred embodiment, the RNA polymerase of the present invention is an RNA polymerase comprising an amino acid sequence having, for example, 90% or more, preferably 95% or more, 96% or more, 97% or more, 98% or more, or 99% or more sequence identity to the amino acid sequence of SEQ ID NO: 1, wherein at least one selected from the group consisting of amino acids at positions corresponding to positions 61, 156, 189, 165, and 661 of the amino acid sequence of SEQ ID NO: 1 is modified.

In a preferred embodiment, the RNA polymerase of the present invention may be an RNA polymerase comprising an amino acid sequence having, for example, 90% or more, preferably 95% or more, 96% or more, 97% or more, 98% or more, or 99% or more sequence identity to the amino acid sequence of SEQ ID NO: 1, wherein at least one selected from the group consisting of the following (i) to (v) is satisfied:
(i) an amino acid at a position corresponding to position 61 of the amino acid sequence of SEQ ID NO: 1 is glutamic acid, aspartic acid, serine, glycine, or proline;
(ii) an amino acid at a position corresponding to position 156 of the amino acid sequence of SEQ ID NO: 1 is asparagine;
(iii) an amino acid at a position corresponding to position 189 of the amino acid sequence of SEQ ID NO: 1 is asparagine;
(iv) an amino acid at a position corresponding to position 165 of the amino acid sequence of SEQ ID NO: 1 is serine, glutamic acid, arginine, or proline; and
(v) an amino acid at a position corresponding to position 661 of the amino acid sequence of SEQ ID NO: 1 is asparagine.

In a preferred embodiment, the RNA polymerase of the present invention comprises an amino acid sequence having, for example, 90% or more, preferably 95% or more, 96% or more, 97% or more, 98% or more or 99% or more sequence identity to the amino acid sequence of SEQ ID NO: 1, wherein, for example,
(1) an amino acid at a position corresponding to position 61 of the amino acid sequence of SEQ ID NO: 1 is glutamic acid, aspartic acid, serine, glycine, or proline;
(2) an amino acid at a position corresponding to position 156 of the amino acid sequence of SEQ ID NO: 1 is asparagine;
(3) an amino acid at a position corresponding to position 189 of the amino acid sequence of SEQ ID NO: 1 is asparagine;
(4) an amino acid at a position corresponding to position 165 of the amino acid sequence of SEQ ID NO: 1 is serine, glutamic acid, arginine, or proline;
(5) an amino acid at a position corresponding to position 661 of the amino acid sequence of SEQ ID NO: 1 is asparagine;
(6) an amino acid at a position corresponding to position 61 of the amino acid sequence of SEQ ID NO: 1 is glutamic acid, and an amino acid at a position corresponding to position 156 of the amino acid sequence of SEQ ID NO: 1 is asparagine;
(7) an amino acid at a position corresponding to position 61 of the amino acid sequence of SEQ ID NO: 1 is glutamic acid, and an amino acid at a position corresponding to position 165 of the amino acid sequence of SEQ ID NO: 1 is serine;
(8) an amino acid at a position corresponding to position 61 of the amino acid sequence of SEQ ID NO: 1 is glutamic acid, and an amino acid at a position corresponding to position 189 of the amino acid sequence of SEQ ID NO: 1 is asparagine;
(9) an amino acid at a position corresponding to position 61 of the amino acid sequence of SEQ ID NO: 1 is glutamic acid, and an amino acid at a position corresponding to position 661 of the amino acid sequence of SEQ ID NO: 1 is asparagine;
(10) an amino acid at a position corresponding to position 61 of the amino acid sequence of SEQ ID NO: 1 is proline, and an amino acid at a position corresponding to position 165 of the amino acid sequence of SEQ ID NO: 1 is serine;
(11) an amino acid at a position corresponding to position 61 of the amino acid sequence of SEQ ID NO: 1 is proline, and an amino acid at a position corresponding to position 189 of the amino acid sequence of SEQ ID NO: 1 is asparagine;
(12) an amino acid at a position corresponding to position 61 of the amino acid sequence of SEQ ID NO: 1 is proline, and an amino acid at a position corresponding to position 661 of the amino acid sequence of SEQ ID NO: 1 is asparagine;
(13) an amino acid at a position corresponding to position 61 of the amino acid sequence of SEQ ID NO: 1 is glutamic acid, an amino acid at a position corresponding to position 156 of the amino acid sequence of SEQ ID NO: 1 is asparagine, and an amino acid at a position corresponding to position 165 of the amino acid sequence of SEQ ID NO: 1 is serine;
(14) an amino acid at a position corresponding to position 61 of the amino acid sequence of SEQ ID NO: 1 is glutamic acid, an amino acid at a position corresponding to position 165 of the amino acid sequence of SEQ ID NO: 1 is serine, and an amino acid at a position corresponding to position 189 of the amino acid sequence of SEQ ID NO: 1 is asparagine;
(15) an amino acid at a position corresponding to position 61 of the amino acid sequence of SEQ ID NO: 1 is glutamic acid, an amino acid at a position corresponding to position 165 of the amino acid sequence of SEQ ID NO: 1 is serine, and an amino acid at a position corresponding to position 661 of the amino acid sequence of SEQ ID NO: 1 is asparagine;
(16) an amino acid at a position corresponding to position 61 of the amino acid sequence of SEQ ID NO: 1 is proline, an amino acid at a position corresponding to position 156 of the amino acid sequence of SEQ ID NO: 1 is asparagine, and an amino acid at a position corresponding to position 165 of the amino acid sequence of SEQ ID NO: 1 is serine;
(17) an amino acid at a position corresponding to position 61 of the amino acid sequence of SEQ ID NO: 1 is proline, an amino acid at a position corresponding to position 165 of the amino acid sequence of SEQ ID NO: 1 is serine, and an amino acid at a position corresponding to position 189 of the amino acid sequence of SEQ ID NO: 1 is asparagine;
(18) an amino acid at a position corresponding to position 61 of the amino acid sequence of SEQ ID NO: 1 is proline, an amino acid at a position corresponding to position 165 of the amino acid sequence of SEQ ID NO: 1 is serine, and an amino acid at a position corresponding to position 661 of the amino acid sequence of SEQ ID NO: 1 is asparagine;
(19) an amino acid at a position corresponding to position 61 of the amino acid sequence of SEQ ID NO: 1 is glutamic acid, an amino acid at a position corresponding to position 156 of the amino acid sequence of SEQ ID NO: 1 is asparagine, an amino acid at a position corresponding to position 165 of the amino acid sequence of SEQ ID NO: 1 is serine, and an amino acid at a position corresponding to position 189 of the amino acid sequence of SEQ ID NO: 1 is asparagine;
(20) an amino acid at a position corresponding to position 61 of the amino acid sequence of SEQ ID NO: 1 is glutamic acid, an amino acid at a position corresponding to position 165 of the amino acid sequence of SEQ ID NO: 1 is serine, an amino acid at a position corresponding to position 189 of the amino acid sequence of SEQ ID NO: 1 is asparagine, and an amino acid at a position corresponding to position 661 of the amino acid sequence of SEQ ID NO: 1 is asparagine;
(21) an amino acid at a position corresponding to position 61 of the amino acid sequence of SEQ ID NO: 1 is proline, an amino acid at a position corresponding to position 156 of the amino acid sequence of SEQ ID NO: 1 is asparagine, an amino acid at a position corresponding to position 165 of the amino acid sequence of SEQ ID NO: 1 is serine, and an amino acid at a position corresponding to position 189 of the amino acid sequence of SEQ ID NO: 1 is asparagine;
(22) an amino acid at a position corresponding to position 61 of the amino acid sequence of SEQ ID NO: 1 is proline, an amino acid at a position corresponding to position 165 of the amino acid sequence of SEQ ID NO: 1 is serine, an amino acid at a position corresponding to position 189 of the amino acid sequence of SEQ ID NO: 1 is asparagine, and an amino acid at a position corresponding to position 661 of the amino acid sequence of SEQ ID NO: 1 is asparagine;
(23) an amino acid at a position corresponding to position 61 of the amino acid sequence of SEQ ID NO: 1 is glutamic acid, an amino acid at a position corresponding to position 156 of the amino acid sequence of SEQ ID NO: 1 is asparagine, an amino acid at a position corresponding to position 165 of the amino acid sequence of SEQ ID NO: 1 is serine, an amino acid at a position corresponding to position 189 of the amino acid sequence of SEQ ID NO: 1 is asparagine, and an amino acid at a position corresponding to position 661 of the amino acid sequence of SEQ ID NO: 1 is asparagine; or
(24) an amino acid at a position corresponding to position 61 of the amino acid sequence of SEQ ID NO: 1 is proline, an amino acid at a position corresponding to position 156 of the amino acid sequence of SEQ ID NO: 1 is asparagine, an amino acid at a position corresponding to position 165 of the amino acid sequence of SEQ ID NO: 1 is serine, an amino acid at a position corresponding to position 189 of the amino acid sequence of SEQ ID NO: 1 is asparagine, and an amino acid at a position corresponding to position 661 of the amino acid sequence of SEQ ID NO: 1 is asparagine,
   preferably,
(10) an amino acid at a position corresponding to position 61 of the amino acid sequence of SEQ ID NO: 1 is proline, and an amino acid at a position corresponding to position 165 of the amino acid sequence of SEQ ID NO: 1 is serine.

In one embodiment, the RNA polymerase of the present invention is characteristically capable of reducing the generation of dsRNA in a transcription reaction. The RNA polymerase of the present invention may be an RNA polymerase in which the proportion of dsRNA in the RNA after a transcription reaction is about 90% or less, preferably about 80% or less, about 70% or less, about 60% or less, about 50% or less, about 40% or less, about 30% or less, about 20% or less, or about 10% or less, as compared to the corresponding wild-type RNA polymerase.

### Method for measuring the proportion of dsRNA in RNA after transcription reaction

Specifically, the proportion of dsRNA in the RNA after a transcription reaction can be confirmed by the following measurement method: 1 µL of an RNA polymerase (50 U/µL) is added to 49 µL of a reaction mixture (the final concentration in the 50 µL of the reaction mixture after enzyme addition is shown below) (40 mM Tris-HCl (pH 8.0), 50 mM NaCl, 8 mM MgCl₂, 5 mM DTT, 10 ng/µL template DNA, 0.4 mM ATP, 0.4 mM CTP, 0.4 mM GTP, 0.4 mM UTP, 0.4 U/µL RNase inhibitor), followed by a reaction at 37°C for 1 hour using a heat block. As a template DNA, dsDNA is used which contains, in the stated order from the 5' end, a promoter corresponding to the RNA polymerase to be measured (one of a T7 promoter, a T3 promoter, an SP6 promoter, and the like), a 5' UTR, a 3' UTR, and a poly(A) (100 nt), and which also contains a firefly luciferase gene (Fluc) as a coding sequence (CDS) between the 5' UTR and the 3' UTR. After the reaction, 2.5 µL of Turbo (registered trademark) DNase (2 U/µL) (available from Thermo Fisher Scientific) is added, followed by an additional reaction at 37°C for 15 minutes. The resulting reaction mixture is purified using a Monarch (registered trademark) RNA Cleanup Kit (available from NEB), whereby a purified RNA is obtained. The absorbance at 260 nm (A260) of the obtained purified RNA is measured. The RNA concentration is calculated, assuming that the RNA concentration is 40 ng/µL when A260 is 1. Using a double-stranded RNA (dsRNA) ELISA kit (K1 based) (available from Exalpha Biologicals), a calibration curve is prepared from measured values obtained by performing ELISA on the dsRNA control included in the kit. The purified RNA is diluted to 100 ng/µL, followed by further dilution to several concentrations. Subsequently, ELISA is performed using a double-stranded RNA (dsRNA) ELISA kit (K1 based). Among the obtained measurement values, measurement values falling within the range of the calibration curve are used, and the dsRNA concentration is calculated from the calibration curve prepared above. Further, the dsRNA concentration is divided by the RNA concentration, whereby the proportion of dsRNA in the RNA (%) (w/w) can be calculated.

In one embodiment, the RNA polymerase of the present invention may exhibit a high specific activity. The RNA polymerase of the present invention may exhibit a specific activity that is about 1.1 times or more, preferably about 1.2 times or more, about 1.3 times or more, about 1.4 times or more, about 1.5 times or more, about 1.6 times or more, about 1.7 times or more, or about 1.8 times or more, higher than that of the corresponding wild-type RNA polymerase. Specifically, the specific activity can be confirmed by the measurement method described below.

### Activity measurement method

The RNA polymerase activity can be measured by the following procedure. The measurement conditions may be appropriately set by a person skilled in the art. For example, when the enzymatic activity is excessively high, the measurement may be performed after appropriately diluting a sample containing the analyte.

First, 45 µL of a reaction mixture (the final concentration in the 50 µL of the reaction mixture after enzyme addition is shown below) (40 mM Tris-HCl (pH 8.0), 50 mM NaCl, 8 mM MgCl₂, 5 mM DTT, 10 ng/µL template DNA, 0.4 mM ATP, 0.4 mM CTP, 0.4 mM GTP, and 0.4 mM UTP) is added to a 0.2 mL PCR tube. Subsequently, 5 µL of a wild-type RNA polymerase with known activity is further added, followed by a reaction at 37°C for 10 minutes using a heat block. After 10 minutes, the reaction is terminated by adding 10 µL of 1 M EDTA. This reaction is performed using a RNA polymerase dilution series prepared. Subsequently, the amount of RNA in each reaction mixture is quantified using a Qubit (registered trademark) RNA BR Assay Kit (available from Thermo Fisher Scientific), and a calibration curve is generated based on the activity values of the RNA polymerase added in each dilution series and the quantified amounts of RNA. The same reaction is also performed for the RNA polymerase to be measured, and its activity is calculated from the obtained amount of RNA using the calibration curve prepared above.

### Measurement of specific activity

The absorbance at 280 nm (A280) is measured, and the enzyme concentration is calculated, assuming that the enzyme concentration is 1 mg/ mL when A280 is 1. The activity value calculated as described above is divided by the enzyme concentration, whereby the specific activity is calculated.

In one embodiment, the RNA polymerase of the present invention may exhibit a higher heat stability than the corresponding wild-type RNA polymerase. In a preferred embodiment, for example, the RNA polymerase of the present invention may be an RNA polymerase that exhibits a residual activity rate of more than 50%, 60% or more, 70% or more, or 80% or more, when heat-treated at 50°C for 5 minutes. In a specific embodiment, the RNA polymerase of the present invention may be an RNA polymerase that exhibits a residual activity rate of 90% or more when heat-treated at 50°C for 5 minutes.

From a further different perspective, for example, the RNA polymerase of the present invention may be a mutant RNA polymerase that exhibits a higher residual activity rate than the corresponding wild-type RNA polymerase when heat-treated at predetermined conditions. Specifically, for example, the RNA polymerase of the present invention may be an RNA polymerase that exhibits a high residual activity rate that is about 1.1 times or more, preferably about 1.2 times or more, about 1.3 times or more, about 1.4 times or more, about 1.5 times or more, about 1.6 times or more, about 1.7 times or more, about 1.8 times or more, or about 1.9 times or more, higher than that of the corresponding wild-type RNA polymerase, when heat-treated at 50°C for 5 minutes.

### Measurement of thermal stability (residual activity rate after heat treatment)

Specifically, the thermal stability can be confirmed by the following measurement method.

First, each mutant RNA polymerase to be measured is diluted to 50 U/µL with a storage buffer (20 mM KPO4 (pH 7.7), 100 mM NaCl, 50% glycerol, 0.1 mM EDTA, 5 mM DTT, and 0.01% Triton (registered trademark) X-100). Subsequently, the activity value before storage is measured according to the procedure described in the activity measurement method. Then, each mutant RNA polymerase to be measured that has been diluted with the storage buffer is stored in a 50°C incubator for 5 minutes. After storage, the activity value after storage is measured in the same manner as before storage, according to the procedure described in the activity measurement method. Then, as described in the following Equation I, the activity value after storage is divided by the activity value before storage, whereby the residual activity rate can be calculated. 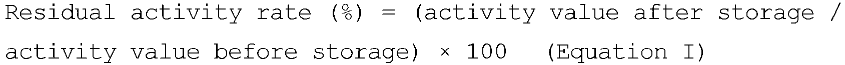 Residual activity rate (%) = (activity value after storage / activity value before storage) × 100 (Equation I)

In another embodiment, the present invention provides a polynucleotide encoding the RNA polymerase of the present invention described above. Such a polynucleotide can be suitably used for expression of the RNA polymerase of the present invention, and/or preparation of a vector for expression thereof, and/or preparation of transformed cells, for example. Here, the term "polynucleotide encoding RNA polymerase" refers to a polynucleotide from which the RNA polymerase protein of the present invention is obtained when expressed by a conventional method. In other words, it refers to a polynucleotide comprising a base sequence corresponding to the amino acid sequence of the RNA polymerase protein of the present invention. A person skilled in the art can readily determine a base sequence corresponding to a predetermined amino acid sequence according to a codon table or the like that is well known in the art. In addition, the polynucleotide encoding the RNA polymerase of the present invention also encompasses a polynucleotide differing due to codon degeneracy. The polynucleotide can be any nucleic acid such as DNA, RNA, etc., but is preferably DNA. The polynucleotide can be synthesized, for example, by artificial gene synthesis. Non-limiting examples of methods for synthesizing DNA by artificial gene synthesis include one that involves chemically synthesizing two or more oligoDNAs of 10 to 100 nt having partial overlap sequences, assembling the synthesized products using the polymerase chain assembly (PCA) method or other enzyme-based methods, designing primers at both ends of the final target sequence, and performing PCR to obtain a double-stranded DNA. In the case of artificial gene synthesis, for example, RNA can be synthesized by synthesizing a double-stranded DNA so as to contain any promoter sequence (a T7 promoter, a T3 promoter, an SP6 promoter, etc.) at the 5' end during the synthesis of the double-stranded DNA described above, and performing an in vitro transcription reaction using the DNA as a template and an RNA polymerase (T7 RNA polymerase, T3 RNA polymerase, SP6 RNA polymerase, etc.) that specifically recognizes any promoter.

In another embodiment, the present invention provides a vector comprising the polynucleotide described above. Such a vector can be suitably used for expression of the RNA polymerase of the present invention and/or preparation of transformed cells, for example. The vector can be prepared by introducing a polynucleotide encoding the RNA polymerase into a vector (e.g., an expression vector, a cloning vector, etc.). The polynucleotide can be introduced into a vector by any method, examples of which include methods using restriction enzyme treatment and ligation, and seamless cloning methods such as Gibson assembly. In a specific embodiment, the vector of the present invention may further comprise elements, such as a promoter, terminator, ribosome binding site, and drug resistance gene, each of which functions in a host cell. Examples of drug resistance genes include genes conferring resistance to drugs, such ampicillin, kanamycin, and tetracycline. In a specific embodiment, the vector of the present invention may comprise an element capable of homologous recombination with the genome of a host cell. The vector is not limited as long as it enables cloning and/or expression of the RNA polymerase of the present invention. Examples include plasmids. Non-limiting examples of plasmids include pUC118, pUC18, pBR322, pBlueScript, pLED-M1, p73, pGW7, and pkk223-3.

In another embodiment, the present invention provides a cell transformed with the vector described above. Such a cell can be suitably used for expression of a protein encoding the RNA polymerase of the present invention. In a specific preferred embodiment, the recombinant host cell of the present invention can be obtained by transforming a host cell with the expression vector described above. For example, the host cell may be *Escherichia coli,* yeast, or the like. *Escherichia coli* is particularly preferred. Examples of *Escherichia coli* include *Escherichia coli* DH5α, JM109, HB101, XL1Blue, PR1, and BL21. In other words, in the present invention, preferably, a gene encoding the RNA polymerase is inserted into the vector described above to obtain an expression vector, and a host cell is further transformed with the expression vector.

In another embodiment, the present invention also provides a method for producing the RNA polymerase described above using the polynucleotide described above, the vector described above, the recombinant host cell described above, and/or one or more. In a specific embodiment, after transforming a host cell with the vector, the resulting recombinant host cell is cultured using any culture apparatus. Non-limiting examples of culture apparatuses include test tubes, flasks, and jar fermenters. Non-limiting examples of media for use in culturing include an LB medium, a 2×YT medium, and a TB medium. Culture conditions can be appropriately set. The culture temperature can be 10 to 40°C, and the culture time can be 1 to 100 hours. Aeration and/or agitation may be performed during culturing, and the culturing may be performed by either batch culture or fedbatch culture. After culturing, the cells are collected by centrifugation or the like, and a crude enzyme solution is extracted by disrupting or lysing the collected cells. Any known method may be used for disrupting or lysing the cells. Non-limiting examples of disruption methods include physical disruption methods, such as ultrasonication, French press, and glass bead disruption. Non-limiting examples of lysing methods include methods using an enzyme such as lysozyme. Any method may be used to obtain a purified RNA polymerase from the crude enzyme solution obtained. For example, the RNA polymerase of the present invention can be isolated by centrifugation, ultracentrifugation, ultrafiltration, nucleic acid removal treatment, salting-out, dialysis, or various types of column chromatography (ion-exchange column chromatography, hydrophobic chromatography, affinity chromatography, gel filtration column chromatography, etc.).

In another embodiment, the present invention also provides a reagent comprising the RNA polymerase described above, the polynucleotide described above, the vector described above, the recombinant host cell described above, and/or one or more of these. In a specific embodiment, the reagent may be a liquid or a solid obtained by removing the moisture by a technique such as lyophilization. In a specific embodiment, the reagent may be placed in any container. In a specific embodiment, the reagent may be divided into any number (one or more) of containers. In a specific embodiment, the reagent may contain a substance that enables expression of the RNA polymerase from the polynucleotide, the vector, and the recombinant host cell. Non-limiting examples of such substances include cell-free protein synthesis reagents. Non-limiting examples of cell-free protein synthesis reagents include those using a cell extract and reconstituted cell-free protein synthesis systems prepared by purifying and mixing factors involved in a translation reaction. Non-limiting examples of substances contained in the cell-free protein synthesis reagent include cell extracts, purified factors involved in translation, 20 amino acids, ATP, GTP, phosphocreatine, creatine kinase, buffers (Tris buffer, phosphate buffer, Good's buffers, etc.), salts (potassium acetate and magnesium acetate), spermidine, and dithiothreitol. Non-limiting examples of reagents using a cell extract include "Mujisaibokun" (registered trademark) available from Taiyo Nippon Sanso Corporation, and those derived from extracts of rabbit reticulocytes, wheat germ, insect cells, *Escherichia coli,* and human cells. Non-limiting examples of reconstituted cell-free protein synthesis systems include PUREfrex (registered trademark) 1.0 available from GeneFrontier Corporation. In a specific embodiment, the reagent may contain a substance necessary for an in vitro transcription reaction. Non-limiting examples of substances required for an in vitro transcription reaction include buffers (Tris buffer, phosphate buffer, Good's buffers, etc.), ribonucleotides (ATP, CTP, GTP, UTP, modified bases (e.g., bases modified by deamination, methylation, and methoxylation, pseudouridylation), etc.), cap analogs (ARCA, mCAP, CleanCap (registered trademark) Reagent AG, CleanCap (registered trademark) Reagent M6, CleanCap (registered trademark) Reagent AU (available from TriLink BioTechnologies), and the like), RNase inhibitors, pyrophosphatases, salts such as NaCl, metal ions such as magnesium ions, EDTA, dithiothreitol, glycerol, surfactants, spermidine, and linear or circular DNA. In a specific embodiment, the reagent may further contain a substance required for post-transcriptional capping. Non-limiting examples of substances required for post-transcriptional capping include capping enzymes and GTP, mRNA Cap 2'-O-methyltransferase, S-adenosylmethionine (SAM), buffers (Tris buffer, phosphate buffer, Good's buffers, etc.), salts such as NaCl and KCl, metal ions such as magnesium ions, EDTA, dithiothreitol, glycerol, and surfactants. In a specific embodiment, the reagent may further contain a substance required for adding a poly(A) to the 3' end of RNA. Non-limiting examples of substances required for adding a poly(A) include ATP, poly(A) polymerase, buffers (Tris buffer, phosphate buffer, Good's buffers, etc.), salts such as NaCl, metal ions such as magnesium ions and manganese ions, EDTA, dithiothreitol, glycerol, and surfactants. In a specific embodiment, the reagent may further contain a substance required for preparing a template DNA for an in vitro transcription reaction. Non-limiting examples of substances required for preparing a template DNA for an in vitro transcription reaction include restriction enzymes, buffers (Tris buffer, phosphate buffer, Good's buffers, etc.), salts such as NaCl, metal ions such as magnesium ions, EDTA, dithiothreitol, glycerol, surfactants, PCR enzymes, and oligoDNAs. In a specific embodiment, the reagent may further contains a substance required for purifying a template DNA for an in vitro transcription reaction. Non-limiting examples of substances required for purifying a template DNA for an in vitro transcription reaction include DNA purification beads and DNA purification columns. In a specific embodiment, the reagent may further contain a substance required for purifying RNA. Non-limiting examples of substances required for purifying RNA include RNA purification beads and RNA purification columns.

The present invention further provides an RNA synthesis method comprising allowing the RNA polymerase described above to act on a template DNA. RNA synthesis is performed by an in vitro transcription reaction. Non-limiting examples of in vitro transcription reactions include a method for synthesizing RNA using the RNA polymerase, with linear or circular DNA as a template. Non-limiting examples of in vitro transcription reactions include a method for synthesizing RNA using the RNA polymerase in the presence of a buffer and ribonucleotides, with linear or circular DNA as a template. Although not particularly limited, the RNA polymerase can be added during the reaction at a concentration ranging from 0.1 U/µL to 20 U/µL, for example. Although not particularly limited, a linear or circular DNA can be added at a concentration ranging from 1 ng/µL to 200 ng/µL, for example. Non-limiting examples of ribonucleotides for use in the in vitro transcription reaction include ATP, CTP, GTP, UTP, and modified bases, such as those modified by deamination, methylation, methoxylation, and pseudouridylation. Although not particularly limited, a ribonucleotide can be added at a concentration ranging from 0.1 mM to 20 mM, for example. Although not particularly limited, cap analogs, such as ARCA, mCAP, CleanCap (registered trademark) Reagent AG, CleanCap (registered trademark) Reagent M6, and CleanCap (registered trademark) Reagent AU (available from TriLink BioTechnologies), may be further added during the in vitro transcription reaction. Although not particularly limited, a cap analog can be added at a concentration ranging from 0.1 mM to 20 mM, for example. Non-limiting examples of buffers for use in the in vitro transcription reaction include Tris buffer, phosphate buffer, and Good's buffers. Although not particularly limited, a buffer can be added at a concentration ranging from 5 mM to 500 mM, for example. Although not particularly limited, an RNase inhibitor, a pyrophosphatase, and the like can be further added during the in vitro transcription reaction. Although not particularly limited, an RNase inhibitor can be added at a concentration ranging from 0.1 U/µL to 20 U/µL, for example. Although not particularly limited, a pyrophosphatase can be added at a concentration ranging from 0.0001 U/µL to 0.02 U/µL, for example. Although not particularly limited, salts such as NaCl, metal ions such as magnesium ions, and the like can be further added during the in vitro transcription reaction. Although not particularly limited, NaCl can be added at a concentration ranging from 1 mM to 200 mM, for example. Although not particularly limited, magnesium ions can be added at a concentration ranging from 0.1 mM to 20 mM, for example. Although not particularly limited, EDTA, dithiothreitol, glycerol, a surfactant, spermidine, and the like can be further added during the in vitro transcription reaction. Although not particularly limited, EDTA can be added at a concentration ranging from 0.0001 mM to 2 mM, for example. Although not particularly limited, dithiothreitol can be added at a concentration ranging from 0.1 mM to 20 mM, for example. Although not particularly limited, glycerol can be added at a concentration ranging from 0.1% to 20%, for example. Although not particularly limited, a surfactant can be added at a concentration ranging from 0.0001% to 1%, for example. Although not particularly limited, spermidine can be added at a concentration ranging from 0.1 mM to 20 mM, for example.

In a specific embodiment, the present invention further provides an RNA synthesis method, wherein the proportion of dsRNA in the synthesized RNA by the RNA synthesis method is less than 90% relative to the proportion of dsRNA in RNA synthesized using a corresponding wild-type RNA polymerase under the same conditions. The proportion may be preferably 80% or less, 70% or less, 60% or less, 50% or less, 40% or less, 30% or less, 25% or less, 20% or less, 15% or less, 13% or less, 10% or less, 9% or less, 8% or less, 7% or less, 6% or less, 5% or less, 4% or less, 3% or less, 2% or less, or 1% or less. The proportion can be calculated by, for example, performing RNA synthesis, under the same conditions, using the corresponding wild-type RNA polymerase and each mutant RNA polymerase to be measured, and determining the proportion of dsRNA in the resulting RNA for each, according to the method described in the section entitled "Method for measuring the proportion of dsRNA in RNA after a transcription reaction," and then comparing the resulting values.

In a specific embodiment, the present invention further provides an RNA synthesis method, wherein the amount of RNA synthesized by the RNA synthesis method (the RNA yield) is at least 110% or more relative to the amount of RNA synthesized using a corresponding wild-type RNA polymerase under the same conditions. The proportion may be preferably 120% or more, 130% or more, 140% or more, 150% or more, 160% or more, 170% or more, 180% or more, or 190% or more. The proportion can be calculated by, for example, performing, under the same conditions, the procedure up to the step of measuring the concentrations of purified RNA for the corresponding wild-type RNA polymerase and each mutant RNA polymerase to be measured, according to the method described in the section entitled "Method for measuring the proportion of dsRNA in RNA after a transcription reaction," calculating the RNA yield by multiplying the RNA concentration by the volume of the purified RNA solution, and then comparing the resulting RNA yields.

In a specific embodiment, the present invention further provides a method for synthesizing mRNA using the RNA polymerase described above. In other words, in the RNA synthesis method, the RNA synthesis may be mRNA synthesis. Non-limiting examples of methods for synthesizing mRNA include a method for synthesizing mRNA using the RNA polymerase in the presence of a buffer and ribonucleotides, with linear or circular DNA as a template. Although not particularly limited, the RNA polymerase can be added during the reaction at a concentration ranging from 0.1 U/µL to 20 U/µL, for example. Although not particularly limited, a linear or circular DNA can be added at a concentration ranging from 1 ng/µL to 200 ng/µL, for example. Non-limiting examples of ribonucleotides for use in mRNA synthesis include ATP, CTP, GTP, UTP, and modified bases, such as those modified by deamination, methylation, methoxylation, and pseudouridylation. Although not particularly limited, a ribonucleotide can be added at a concentration ranging from 0.1 mM to 20 mM, for example. Non-limiting examples of buffers for use in mRNA synthesis include Tris buffer, phosphate buffer, and Good's buffers. Although not particularly limited, a buffer can be added at a concentration ranging from 5 mM to 500 mM, for example. Although not particularly limited, an RNase inhibitor, a pyrophosphatase, and the like can be further added during mRNA synthesis. Although not particularly limited, an RNase inhibitor can be added at a concentration ranging from 0.1 U/µL to 20 U/µL, for example. Although not particularly limited, a pyrophosphatase can be added at a concentration ranging from 0.0001 U/µL to 0.02 U/µL, for example. Although not particularly limited, salts such as NaCl, metal ions such as magnesium ions, and the like can be further added during mRNA synthesis. Although not particularly limited, NaCl can be added at a concentration ranging from 1 mM to 200 mM, for example. Although not particularly limited, magnesium ions can be added at a concentration ranging from 0.1 mM to 20 mM, for example. Although not particularly limited, EDTA, dithiothreitol, glycerol, a surfactant, spermidine, and the like can be further added during mRNA synthesis. Although not particularly limited, EDTA can be added at a concentration ranging from 0.0001 mM to 2 mM, for example. Although not particularly limited, glycerol can be added at a concentration ranging from 0.1% to 20%, for example. Although not particularly limited, a surfactant can be added at a concentration ranging from 0.0001% to 1%, for example. Although not particularly limited, spermidine can be added at a concentration ranging from 0.1 mM to 20 mM, for example. mRNA includes a cap structure at its 5' end. Non-limiting examples of methods for adding the cap structure include post-transcriptional capping and co-transcriptional capping. Non-limiting examples of post-transcriptional capping include a method including adding Cap-0 to the 5' end of mRNA using a capping enzyme, GTP, and SAM, and further converting Cap-0 into Cap-1 using an mRNA Cap 2'-O-methyltransferase and SAM, as necessary. Non-limiting examples of co-transcriptional capping include a method using cap analogs, such as ARCA, mCAP, CleanCap (registered trademark) Reagent AG, CleanCap (registered trademark) Reagent M6, and CleanCap (registered trademark) Reagent AU (available from TriLink BioTechnologies)). Although not particularly limited, a cap analog can be added at a concentration ranging from 0.1 mM to 20 mM, for example. mRNA includes a poly(A) at its 3' end. When the template DNA does not include a poly(A), non-limiting examples of methods for adding a poly(A) to the 3' end of the synthesized RNA include a method using poly(A) polymerase.

In a specific embodiment, the present invention further provides an mRNA synthesis method, wherein the proportion of dsRNA in the synthesized mRNA is less than 90% relative to the proportion of dsRNA in mRNA synthesized using a corresponding wild-type RNA polymerase under the same conditions. The proportion may be preferably 80% or less, 70% or less, 60% or less, 50% or less, 40% or less, 30% or less, 20% or less, or 10% or less. The proportion can be calculated by, for example, performing mRNA synthesis, under the same conditions, using the corresponding wild-type RNA polymerase and each mutant RNA polymerase to be measured, and determining the proportion of dsRNA in the resulting mRNA for each, according to the method described in the section entitled "Method for measuring the proportion of dsRNA in RNA after a transcription reaction," and then comparing the resulting values.

In a specific embodiment, the present invention further provides an mRNA synthesis method, wherein the amount of mRNA synthesized by the mRNA synthesis method (mRNA yield) is at least 110% or more relative to the amount of mRNA synthesized using a corresponding wild-type RNA polymerase under the same conditions. The proportion may be preferably 120% or more, 130% or more, 140% or more, 150% or more, 160% or more, 170% or more, 180% or more, or 190% or more. The proportion can be calculated by, for example, performing, under the same conditions, the procedure up to the step of measuring the concentrations of purified mRNA for the corresponding wild-type RNA polymerase and each mutant RNA polymerase to be measured, according to the method described in the section entitled "Method for measuring the proportion of dsRNA in RNA after a transcription reaction," calculating the mRNA yield by multiplying the mRNA concentration by the volume of the purified mRNA solution, and then comparing the resulting mRNA yields.

As a specific embodiment, the present invention further provides a method for synthesizing a non-coding RNA using the RNA polymerase described above. In other words, in the RNA synthesis method, the RNA synthesis may be non-coding RNA synthesis. Non-limiting examples of non-coding RNAs include microRNA, siRNA, piRNA, rRNA, tRNA, snRNA, snoRNA, SLRNA, SRPRNA, mRNA-like non-coding RNA, and oligonucleotides. Non-limiting examples of methods for synthesizing non-coding RNA include a method using a template DNA containing DNA corresponding to the target non-coding RNA. The template DNA is not limited as long as it contains DNA corresponding to the target non-coding RNA. However, preferably it contains, upstream of the corresponding DNA, a promoter (a T7 promoter, a T3 promoter, an SP6 promoter, etc.) sequence recognized by the RNA polymerase of the present invention. The template DNA may be prepared by any method. Non-limiting examples of methods include a method for treating a plasmid with a restriction enzyme and a method using DNA synthesized by artificial gene synthesis. The plasmid is not limited as long as it contains DNA corresponding to the target non-coding RNA. It may be one containing the above-described promoter sequence, a restriction enzyme site, a drug resistance gene, and the like. Non-limiting examples of methods for obtaining the plasmid include a method including: synthesizing DNA corresponding to the target non-coding RNA by artificial gene synthesis; cloning the DNA into a plasmid containing, upstream of the site into which the above DNA is inserted, a promoter (a T7 promoter, a T3 promoter, an SP6 promoter, etc.) sequence recognized by the RNA polymerase of the present invention and, downstream thereof, a restriction enzyme site, and optionally containing a drug resistance gene or the like at any position; transforming the plasmid into a host, preferably *Escherichia coli,* by any method; culturing the resulting recombinant host cells by any method; and extracting the plasmid. The resulting plasmid can be linearized by restriction enzyme treatment using any restriction enzyme capable of cleaving the restriction enzyme site contained in the above plasmid, and can be used as a template DNA after purification by any method. Non-limiting examples of methods using DNA synthesized by artificial gene synthesis include a method including: synthesizing, by artificial gene synthesis, a double stranded DNA containing DNA corresponding to the target non-coding RNA and, upstream thereof, a promoter (a T7 promoter, a T3 promoter, an SP6 promoter, etc.) sequence recognized by the RNA polymerase of the present invention; amplifying the resulting double-stranded DNA by PCR or the like, if necessary; and further purifying the amplified double-stranded DNA, if necessary, to obtain a template DNA.

In a specific embodiment, the present invention further provides a method for synthesizing a guide RNA for gene editing, using the RNA polymerase described above. Non-limiting examples of methods for gene editing include the CRISPER/Cas9 system.

In a specific embodiment, the present invention further provides a method using the RNA polymerase described above for synthesizing an RNA therapeutic. Non-limiting examples of RNA therapeutics include those using mRNA, siRNA, microRNA, antisense RNA, or an aptamer. Non-limiting examples of methods using the RNA polymerase for synthesizing an RNA therapeutic include a method including performing an in vitro transcription reaction by the method described above using the RNA polymerase and a template DNA containing DNA corresponding to any RNA (e.g., mRNA, siRNA, microRNA, antisense RNA, an aptamer, etc.) for use in an RNA therapeutic. The template DNA containing DNA corresponding to any RNA (e.g., mRNA, siRNA, microRNA, antisense RNA, an aptamer, etc.) is not limited as long as it is any DNA containing DNA corresponding to any RNA. It may be one containing, upstream of the corresponding DNA, a promoter (a T7 promoter, a T3 promoter, an SP6 promoter, etc.) sequence recognized by the RNA polymerase of the present invention, and optionally, a 5' UTR and, downstream thereof, a 3' UTR, a poly(A), a restriction enzyme site, and the like. The template DNA may be prepared by any method, such as a method known to a person skilled in the art. For example, the methods described above for synthesizing a non-coding RNA may be used with appropriate modifications.

In a specific embodiment, the present invention further provides a method using the RNA polymerase described above in an isothermal amplification reaction. Non-limiting examples of isothermal amplification reactions techniques include NASBA and TMA methods.

In a specific embodiment, the present invention further provides a method for performing genetic testing using the RNA polymerase described above. Non-limiting examples of methods for performing genetic testing include a method including confirming the presence or absence of a pathogen by amplifying and detecting a gene of the target pathogen by the above-described isothermal amplification reaction or the like.

In a specific embodiment, the present invention further provides a method for performing in vivo protein expression or in vitro cell-free protein synthesis using the RNA polymerase described above. Non-limiting examples of methods for performing in vivo protein expression using the RNA polymerase include a method including transfecting, by any method, a cell with mRNA synthesized using the RNA polymerase. In a specific embodiment, mRNA is not limited as long as it enables expression of the target protein. Preferably, it contains RNA encoding the target protein. It may be one containing a 5' UTR upstream thereof and a 3' UTR, a poly(A), and the like downstream thereof. In a specific embodiment, although not particularly limited, mRNA can be synthesized by a method including performing an in vitro transcription reaction, for example by the method described above, using, as a template, a template DNA containing DNA encoding the target protein. The template DNA may be prepared by any method, such as a method known to a person skilled in the art. For example, the methods described above for synthesizing a non-coding RNA may be used with appropriate modifications. In a specific embodiment, although not particularly limited, when performing protein expression, culturing and the like may be performed after transfection, if necessary. Non-limiting examples of methods for performing in vitro cell-free protein synthesis using the RNA polymerase described above include a method including adding the RNA polymerase described above, the polynucleotide described above, the vector described above, the recombinant host cell described above, and/or one or more of these to a cell-free protein synthesis reagent.

### Examples

The present invention is specifically described below with reference to the examples; however, the present invention is not limited to the following examples.

### Example 1: Construction of plasmid for T7 RNA polymerase expression

A gene (SEQ ID NO: 2) encoding wild-type T7 RNA polymerase with a 6×His tag at the N-terminus was artificially synthesized and cloned into the EcoRI-PstI cloning site of pkk223-3, and a plasmid incorporating a gene encoding wild-type T7 RNA polymerase with a 6×His tag at the N-terminus was constructed. The thus-constructed expression plasmid is referred to below as "pkk223-3-T7RNAP." Expression plasmids for each modified T7 RNA polymerase were constructed by site-directed mutagenesis using pkk223-3-T7RNAP as a template, the primers shown in Table 1, and a KOD-Plus Mutagenesis Kit (available from Toyobo Co., Ltd.). Expression plasmids for each modified T7 RNA polymerase with multiple modified amino acids were constructed by repeating site-directed mutagenesis by the same method, using, as a template, an expression plasmid for a modified T7 RNA polymerase, which was obtained by site-directed mutagenesis.

**Table1**

| List of primers used for mutagenesis | | |
|---|---|---|
| Mutation | Fwd primer SEQ ID NO: | Rev primer SEQ ID NO: |
| **A61E** | **3** | **4** |
| **A61D** | **5** | **4** |
| **A61S** | **6** | **4** |
| **A61G** | **7** | **4** |
| **A61P** | **8** | **4** |
| **D156N** | **9** | **10** |
| **N165S** | **11** | **12** |
| **N165E** | **13** | **12** |
| **N165R** | **14** | **12** |
| **N165P** | **15** | **12** |
| **D189N** | **16** | **17** |
| **S661N** | **18** | **19** |

The obtained plasmids each have a base sequence encoding a modified T7 RNA polymerase with a 6×His tag at the N-terminus.

### Example 2: Preparation of T7 RNA polymerase

Wild-type T7 RNA polymerase with a 6×His tag at the N-terminus, and modified T7 RNA polymerases were all prepared by the same method. A preparation example of wild-type T7 RNA polymerase is shown below. *Escherichia coli* JM109 was transformed with pkk223-3-T7RNAP, followed by static culture on LB agar medium containing 100 µg/mL of ampicillin at 37°C for 20 hours. A single colony on the agar medium was inoculated into 3 mL of LB liquid medium containing 100 µg/mL of ampicillin, followed by shaking culture in a 15 mL test tube at 37°C for 20 hours. 1 mL of the culture was inoculated into 80 mL of TB liquid medium containing 100 µg/mL of ampicillin, followed by shaking culture in a 500 mL Sakaguchi flask at 37°C for 20 hours. Then, the bacterial cells were collected from the culture by centrifugation. 1 g of the obtained bacterial cells was suspended in 10 mL of lysis buffer (20 mM Tris-HCl (pH 7.5), 500 mM NaCl, 10% glycerol, 1 mM DTT, and 20 mM imidazole), and the bacterial cells were disrupted on ice using an ultrasonic disruptor. The bacterial lysate was centrifuged at 20000 × g at 4°C for 20 minutes to collect the supernatant. Then, the supernatant was purified using His GraviTrap (available from GE HealthCare). An equilibration and wash buffer containing 20 mM Tris-HCl (pH 7.5), 500 mM NaCl, 10% glycerol, 1 mM DTT, and 20 mM imidazole was used. An elution buffer containing 20 mM Tris-HCl (pH 7.5), 500 mM NaCl, 10% glycerol, 1 mM DTT, and 300 mM imidazole was used. The eluted fractions were collected, and the buffer was replaced with a storage buffer (20 mM KPO4 (pH 7.7), 100 mM NaCl, 50% glycerol, 0.1 mM EDTA, 5 mM DTT, and 0.01% Triton (registered trademark) X-100), whereby wild-type T7 RNA polymerase with a 6×His tag at the N-terminus was obtained. In the preparation example described above, transformation was performed using the expression plasmid of each modified T7 RNA polymerase produced in Example 1, whereby each modified T7 RNA polymerase with a 6×His tag at the N-terminus was obtained.

### Example 3: Measurement of the specific activity of T7 RNA polymerase

The wild-type T7 RNA polymerase with a His tag at the N-terminus and each of the modified T7 RNA polymerases similarly with a His tag at the N-terminus (A61E, A61D, A61S, A61P, D156N, N165S, D189N, S661N, A61E/D156N, A61E/N165S, A61E/D189N, A61E/S661N, A61P/N165S, A61P/D189N, and A61P/S661N) were used to measure the activity by the activity measurement method described above. The template DNA used here was 4 kbp dsDNA (SEQ ID NO: 20) containing a T7 promoter sequence. The length downstream of the T7 promoter in this dsDNA was about 1 kbp, and the length of the transcribed RNA was about 1 kb. The protein concentration of each modified T7 RNA polymerase was determined by measuring the absorbance at A280 absorbance, and the specific activity was calculated by dividing the activity by the protein concentration. Table 2 shows the results.

**Table 2**

| Specific activities of various mutants | | |
|---|---|---|
| | Specific activity **(KU/mg)** | Specific activity relative to wild-type |
| **Wild-type** | **149** | **1.00** |
| **A61E** | **279** | **1.87** |
| **A61D** | **316** | **2.12** |
| **A61S** | **254** | **1.70** |
| **A61P** | **292** | **1.96** |
| **D156N** | **208** | **1.40** |
| **N165S** | **234** | **1.57** |
| **D189N** | **201** | **1.35** |
| **S661N** | **231** | **1.55** |
| **A61E/D156N** | **356** | **2.39** |
| **A61E/N165S** | **402** | **2.70** |
| **A61E/D189N** | **450** | **3.02** |
| **A61E/S661N** | **399** | **2.68** |
| **A61P/N165S** | **377** | **2.53** |
| **A61P/D189N** | **296** | **1.99** |
| **A61P/S661N** | **320** | **2.15** |

The results in Table 2 confirmed that the specific activity was improved in all modified T7 RNA polymerases as compared to in the wild-type.

### Example 4: Measurement of the heat resistance of T7 RNA polymerase

The wild-type T7 RNA polymerase with a His tag at the N-terminus and each of the modified T7 RNA polymerases similarly with a His tag at the N-terminus (A61E, D156N, N165S, D189N, and S661N) were each diluted to 50 U/µL with a storage buffer (20 mM KPO4 (pH 7.7), 100 mM NaCl, 50% glycerol, 0.1 mM EDTA, 5 mM DTT, and 0.01% Triton (registered trademark) X-100). Then, the activity value before storage was measured according to the procedure described in the activity measurement method. Each T7 RNA polymerase diluted with the storage buffer was heat-treated in a 50°C incubator for 5 minutes. Then, the activity was measured, and the residual activity rate was calculated from the activity values before and after storage. Table 3 shows the results.

**Table 3**

| Residual activity rates of various mutants | |
|---|---|
| | Residual activity (%) after heat treatment at 50°C for 5 min |
| Wild-type | 50 |
| A61E | 81 |
| D156N | 67 |
| N165S | 90 |
| D189N | 108 |
| S661N | 94 |

The results in Table 3 confirmed that the residual activity rate after heat treatment was higher in all modified T7 RNA polymerases than in the wild-type.

### Example 5: Measurement of the amount of dsRNA after transcription reaction

The wild-type T7 RNA polymerase with a His tag at the N-terminus and each of the modified T7 RNA polymerases similarly with a His tag at the N-terminus (A61E, A61D, A61S, A61G, A61P, D156N, N165S, N165E, N165R, N165P, D189N, S661N, A61E/D156N, A61E/N165S, A61E/D189N, A61E/S661N, A61P/N165S, A61P/D189N, A61P/S661N, A61E/D156N/N165S, A61E/N165S/D189N, A61E/N165S/S661N, A61P/D156N/N165S, A61P/N165S/D189N, A61P/N165S/S661N, A61E/D156N/N165S/D189N, A61E/N165S/D189N/S661N, A61P/D156N/N165S/D189N, A61P/N165S/D189N/S661N, A61E/D156N/N165S/D189N/S661N, and A61P/D156N/N165S/D189N/S661N) were used to measure the activity by the same activity measurement method used in Example 3. They were all diluted to 50 U/µL with a storage buffer (20 mM KPO4 (pH 7.7), 100 mM NaCl, 50% glycerol, 0.1 mM EDTA, 5 mM DTT, and 0.01% Triton (registered trademark) X-100). Then, 1 µL of each T7 RNA polymerase (50 U/µL) was added to 49 µL of a reaction mixture (the final concentration in the 50 µL of the reaction mixture after enzyme addition is shown below) (40 mM Tris-HCl (pH 8.0), 50 mM NaCl, 8 mM MgCl₂, 5 mM DTT, 10 ng/µL template DNA, 0.4 mM ATP, 0.4 mM CTP, 0.4 mM GTP, 0.4 mM UTP, 0.4 U/µL RNase inhibitor), followed by a reaction at 37°C for 1 hour using a heat block. The template DNA was dsDNA (SEQ ID NO: 21) containing a T7 promoter, a 5' UTR, a 3' UTR, a poly(A) (100 nt), and a firefly luciferase gene (Fluc) as a coding sequence (CDS). The generated RNA was an about 1.9 kb uncapped mRNA containing a 5' UTR, a CDS (Fluc), a 3' UTR, and a poly(A) in the stated order from the 5' side. After the reaction, 2.5 µL Turbo (registered trademark) DNase (2 U/µL) (available from Thermo Fisher Scientific) was added, followed by an additional reaction at 37°C for 15 minutes. The resulting reaction mixture was purified using a Monarch (registered trademark) RNA Cleanup Kit (available from NEB), whereby a purified RNA was obtained. The absorbance at 260 nm (A260) of the thus-obtained purified RNA was measured. The RNA concentration was calculated, assuming that the RNA concentration is 40 ng/µL when A260 is 1. Using a double-stranded RNA (dsRNA) ELISA kit (K1 based) (available from Exalpha Biologicals), a calibration curve was prepared from measured values obtained by performing ELISA on the dsRNA control included in the kit. Each purified RNA was diluted to 100 ng/µL, followed by dilution to several concentrations. Subsequently, ELISA was performed using a double-stranded RNA (dsRNA) ELISA kit (K1 based). Among the obtained measurement values, measurement values falling within the range of the calibration curve were used, and the dsRNA concentration was calculated from the calibration curve prepared above. Further, the dsRNA concentration was divided by the RNA concentration, whereby the proportion of dsRNA in the RNA (%) (w/w) was calculated. Table 4 shows the results.

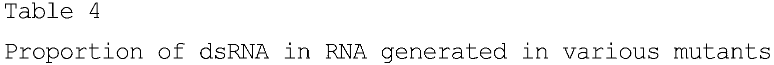

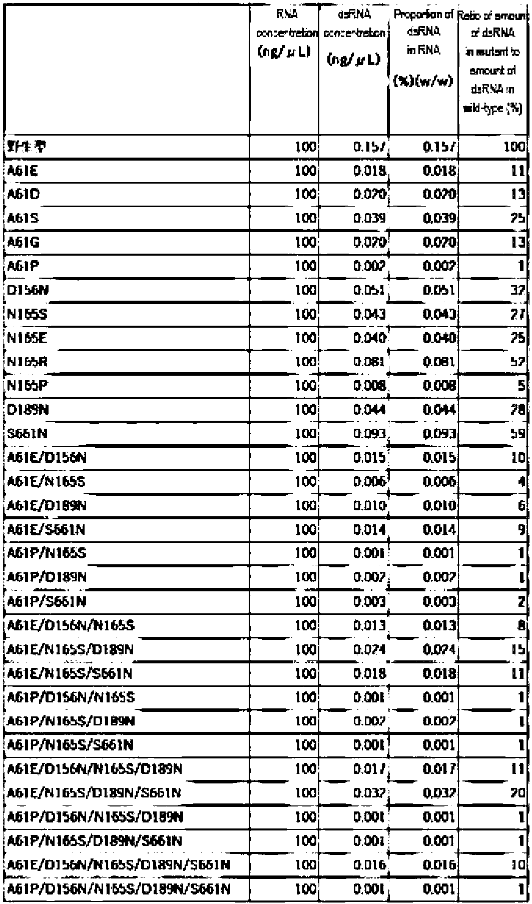

The results in Table 4 confirmed that the ratio of the amount of dsRNA to the amount of RNA after the transcription reaction was lower in all modified T7 RNA polymerases than in the wild-type.

### Industrial Applicability

The present invention is suitably applicable to RNA synthesis for various purposes.

## Claims

1. An RNA polymerase comprising an amino acid sequence having 90% or more sequence identity to the amino acid sequence of SEQ ID NO: 1, wherein at least one selected from the group consisting of amino acids at positions corresponding to positions 61, 156, 189, 165, and 661 of the amino acid sequence of SEQ ID NO: 1 is modified.

2. An RNA polymerase comprising an amino acid sequence having 90% or more sequence identity to the amino acid sequence of SEQ ID NO: 1, wherein at least one selected from the group consisting of the following (i) to (v) is satisfied:
(i) an amino acid at a position corresponding to position 61 of the amino acid sequence of SEQ ID NO: 1 is glutamic acid, aspartic acid, serine, glycine, or proline;
(ii) an amino acid at a position corresponding to position 156 of the amino acid sequence of SEQ ID NO: 1 is asparagine;
(iii) an amino acid at a position corresponding to position 189 of the amino acid sequence of SEQ ID NO: 1 is asparagine;
(iv) an amino acid at a position corresponding to position 165 of the amino acid sequence of SEQ ID NO: 1 is serine, glutamic acid, arginine, or proline; and
(v) an amino acid at a position corresponding to position 661 of the amino acid sequence of SEQ ID NO: 1 is asparagine.

3. The RNA polymerase according to claim 1 or 2, comprising an amino acid sequence having 90% or more sequence identity to the amino acid sequence of SEQ ID NO: 1, wherein
(1) an amino acid at a position corresponding to position 61 of the amino acid sequence of SEQ ID NO: 1 is glutamic acid, aspartic acid, serine, glycine, or proline;
(2) an amino acid at a position corresponding to position 156 of the amino acid sequence of SEQ ID NO: 1 is asparagine;
(3) an amino acid at a position corresponding to position 189 of the amino acid sequence of SEQ ID NO: 1 is asparagine;
(4) an amino acid at a position corresponding to position 165 of the amino acid sequence of SEQ ID NO: 1 is serine, glutamic acid, arginine, or proline;
(5) an amino acid at a position corresponding to position 661 of the amino acid sequence of SEQ ID NO: 1 is asparagine;
(6) an amino acid at a position corresponding to position 61 of the amino acid sequence of SEQ ID NO: 1 is glutamic acid, and an amino acid at a position corresponding to position 156 of the amino acid sequence of SEQ ID NO: 1 is asparagine;
(7) an amino acid at a position corresponding to position 61 of the amino acid sequence of SEQ ID NO: 1 is glutamic acid, and an amino acid at a position corresponding to position 165 of the amino acid sequence of SEQ ID NO: 1 is serine;
(8) an amino acid at a position corresponding to position 61 of the amino acid sequence of SEQ ID NO: 1 is glutamic acid, and an amino acid at a position corresponding to position 189 of the amino acid sequence of SEQ ID NO: 1 is asparagine;
(9) an amino acid at a position corresponding to position 61 of the amino acid sequence of SEQ ID NO: 1 is glutamic acid, and an amino acid at a position corresponding to position 661 of the amino acid sequence of SEQ ID NO: 1 is asparagine;
(10) an amino acid at a position corresponding to position 61 of the amino acid sequence of SEQ ID NO: 1 is proline, and an amino acid at a position corresponding to position 165 of the amino acid sequence of SEQ ID NO: 1 is serine;
(11) an amino acid at a position corresponding to position 61 of the amino acid sequence of SEQ ID NO: 1 is proline, and an amino acid at a position corresponding to position 189 of the amino acid sequence of SEQ ID NO: 1 is asparagine;
(12) an amino acid at a position corresponding to position 61 of the amino acid sequence of SEQ ID NO: 1 is proline, and an amino acid at a position corresponding to position 661 of the amino acid sequence of SEQ ID NO: 1 is asparagine;
(13) an amino acid at a position corresponding to position 61 of the amino acid sequence of SEQ ID NO: 1 is glutamic acid, an amino acid at a position corresponding to position 156 of the amino acid sequence of SEQ ID NO: 1 is asparagine, and an amino acid at a position corresponding to position 165 of the amino acid sequence of SEQ ID NO: 1 is serine;
(14) an amino acid at a position corresponding to position 61 of the amino acid sequence of SEQ ID NO: 1 is glutamic acid, an amino acid at a position corresponding to position 165 of the amino acid sequence of SEQ ID NO: 1 is serine, and an amino acid at a position corresponding to position 189 of the amino acid sequence of SEQ ID NO: 1 is asparagine;
(15) an amino acid at a position corresponding to position 61 of the amino acid sequence of SEQ ID NO: 1 is glutamic acid, an amino acid at a position corresponding to position 165 of the amino acid sequence of SEQ ID NO: 1 is serine, and an amino acid at a position corresponding to position 661 of the amino acid sequence of SEQ ID NO: 1 is asparagine;
(16) an amino acid at a position corresponding to position 61 of the amino acid sequence of SEQ ID NO: 1 is proline, an amino acid at a position corresponding to position 156 of the amino acid sequence of SEQ ID NO: 1 is asparagine, and an amino acid at a position corresponding to position 165 of the amino acid sequence of SEQ ID NO: 1 is serine;
(17) an amino acid at a position corresponding to position 61 of the amino acid sequence of SEQ ID NO: 1 is proline, an amino acid at a position corresponding to position 165 of the amino acid sequence of SEQ ID NO: 1 is serine, and an amino acid at a position corresponding to position 189 of the amino acid sequence of SEQ ID NO: 1 is asparagine;
(18) an amino acid at a position corresponding to position 61 of the amino acid sequence of SEQ ID NO: 1 is proline, an amino acid at a position corresponding to position 165 of the amino acid sequence of SEQ ID NO: 1 is serine, and an amino acid at a position corresponding to position 661 of the amino acid sequence of SEQ ID NO: 1 is asparagine;
(19) an amino acid at a position corresponding to position 61 of the amino acid sequence of SEQ ID NO: 1 is glutamic acid, an amino acid at a position corresponding to position 156 of the amino acid sequence of SEQ ID NO: 1 is asparagine, an amino acid at a position corresponding to position 165 of the amino acid sequence of SEQ ID NO: 1 is serine, and an amino acid at a position corresponding to position 189 of the amino acid sequence of SEQ ID NO: 1 is asparagine;
(20) an amino acid at a position corresponding to position 61 of the amino acid sequence of SEQ ID NO: 1 is glutamic acid, an amino acid at a position corresponding to position 165 of the amino acid sequence of SEQ ID NO: 1 is serine, an amino acid at a position corresponding to position 189 of the amino acid sequence of SEQ ID NO: 1 is asparagine, and an amino acid at a position corresponding to position 661 of the amino acid sequence of SEQ ID NO: 1 is asparagine;
(21) an amino acid at a position corresponding to position 61 of the amino acid sequence of SEQ ID NO: 1 is proline, an amino acid at a position corresponding to position 156 of the amino acid sequence of SEQ ID NO: 1 is asparagine, an amino acid at a position corresponding to position 165 of the amino acid sequence of SEQ ID NO: 1 is serine, and an amino acid at a position corresponding to position 189 of the amino acid sequence of SEQ ID NO: 1 is asparagine;
(22) an amino acid at a position corresponding to position 61 of the amino acid sequence of SEQ ID NO: 1 is proline, an amino acid at a position corresponding to position 165 of the amino acid sequence of SEQ ID NO: 1 is serine, an amino acid at a position corresponding to position 189 of the amino acid sequence of SEQ ID NO: 1 is asparagine, and an amino acid at a position corresponding to position 661 of the amino acid sequence of SEQ ID NO: 1 is asparagine;
(23) an amino acid at a position corresponding to position 61 of the amino acid sequence of SEQ ID NO: 1 is glutamic acid, an amino acid at a position corresponding to position 156 of the amino acid sequence of SEQ ID NO: 1 is asparagine, an amino acid at a position corresponding to position 165 of the amino acid sequence of SEQ ID NO: 1 is serine, an amino acid at a position corresponding to position 189 of the amino acid sequence of SEQ ID NO: 1 is asparagine, and an amino acid at a position corresponding to position 661 of the amino acid sequence of SEQ ID NO: 1 is asparagine; or
(24) an amino acid at a position corresponding to position 61 of the amino acid sequence of SEQ ID NO: 1 is proline, an amino acid at a position corresponding to position 156 of the amino acid sequence of SEQ ID NO: 1 is asparagine, an amino acid at a position corresponding to position 165 of the amino acid sequence of SEQ ID NO: 1 is serine, an amino acid at a position corresponding to position 189 of the amino acid sequence of SEQ ID NO: 1 is asparagine, and an amino acid at a position corresponding to position 661 of the amino acid sequence of SEQ ID NO: 1 is asparagine.

4. A polynucleotide encoding the RNA polymerase according to claim 1.

5. A vector comprising the polynucleotide according to claim 4.

6. A recombinant host cell transformed with the vector according to claim 5.

7. A method for producing an RNA polymerase using the polynucleotide according to claim 4, the vector according to claim 5, and/or the recombinant host cell according to claim 6.

8. A reagent comprising the RNA polymerase according to claim 1, the polynucleotide according to claim 4, the vector according to claim 5, and/or the recombinant host cell according to claim 6.

9. An RNA synthesis method comprising allowing the RNA polymerase according to claim 1 to act on a template DNA.

10. The method according to claim 9, wherein the proportion of dsRNA in the synthesized RNA is less than 90% relative to the proportion of dsRNA in RNA synthesized using a corresponding wild-type RNA polymerase under the same conditions.

11. The method according to claim 9 or 10, wherein the RNA synthesis is mRNA synthesis.

12. The method according to claim 11, wherein the proportion of dsRNA in the synthesized mRNA is less than 90% relative to the proportion of dsRNA in mRNA synthesized using a corresponding wild-type RNA polymerase under the same conditions.

13. A method for synthesizing an RNA therapeutic using the RNA polymerase according to claim 1.
